(19) Europäisches Patentamt
European Patent Office
Office européen des brevets

(11) **EP 4 162 878 A1**

(12) **EUROPÄISCHE PATENTANMELDUNG**

(43) Veröffentlichungstag:
**12.04.2023   Patentblatt 2023/15**

(21) Anmeldenummer: **22200375.8**

(22) Anmeldetag: **07.10.2022**

(51) Internationale Patentklassifikation (IPC):
***A61B 5/397*** (2021.01)        *A61B 5/00* (2006.01)

(52) Gemeinsame Patentklassifikation (CPC):
**A61B 5/397;** A61B 5/4851

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**
Benannte Erstreckungsstaaten:
**BA**
Benannte Validierungsstaaten:
**KH MA MD TN**

(30) Priorität: **08.10.2021   DE 102021126194**

(71) Anmelder: **Fraunhofer-Gesellschaft zur Förderung der angewandten Forschung e.V.
80686 München (DE)**

(72) Erfinder:
• **KUSCHE, Roman
21075 Hamburg (DE)**
• **OLTMANN, Andra
23558 Lübeck (DE)**
• **GRASSHOFF, Jan
23552 Lübeck (DE)**

(74) Vertreter: **Kuttenkeuler, David
SKM-IP PartGmbB
Oberanger 45
80331 München (DE)**

(54) **VERFAHREN ZUR VEKTORIELLEN ERFASSUNG UND VERARBEITUNG ELEKTROMYOGRAPHISCHER SIGNALE**

(57)     Ein Verfahren zur Separation von Nutz- und Störsignalen in einer elektromyografischen (EMG)-Messung und/oder zur Bestimmung des Signal-verursachenden Muskels in einer elektrisch aktiven Region an Muskeln, die Verwendung des Verfahrens zur EMG-Diagnostik, zur Ansteuerung von Prothesen, zum Training von Muskeln, oder zur Steuerung von Computer-Programmen und/oder Computer-Spielen und ein computerimplementiertes Verfahren, ein Computerprogrammprodukt, ein computerlesbares Speichermedium und eine Vorrichtung zur Separation von Nutz- und Störsignalen in einer EMG-Messung und/oder zur Bestimmung des Signal-verursachenden Muskels in einer elektrisch aktiven Region an Muskeln werden bereitgestellt.

FIGUR 2

EP 4 162 878 A1

**Beschreibung**

Die vorliegende Erfindung beschreibt ein Verfahren zur Separation von Nutz- und Störsignalen in einer elektromyografischen (EMG)-Messung und/oder zur Bestimmung des Signal-verursachenden Muskels in einer elektrisch aktiven Region an Muskeln. Die vorliegende Erfindung betrifft auch die Verwendung des erfindungsgemäßen Verfahrens zur EMG-Diagnostik, zur Ansteuerung von Prothesen, zum Training von Muskeln, oder zur Steuerung von Computer-Programmen und/oder Computer-Spielen. Zudem wird ein computerimplementiertes Verfahren, ein Computerprogrammprodukt, ein computerlesbares Speichermedium und eine Vorrichtung zur Separation von Nutz- und Störsignalen in einer EMG-Messung und/oder zur Bestimmung des Signal-verursachenden Muskels in einer elektrisch aktiven Region an Muskeln bereitgestellt.

HINTERGRUND DER ERFINDUNG

Die Elektromyographie (EMG) ist ein Verfahren zur Erfassung der elektrischen Aktivität, wie sie bei der Muskelkontraktion entsteht. Wird ein Muskel erregt, so entsteht in den Muskelfasern ein Aktionspotential, welches sich aufgrund der anisotropen Leitfähigkeitseigenschaften einer Muskelfaser bevorzugt in Faserrichtung ausbreitet. Mit der EMG wird das Summenpotential aller aktiven Muskelfasern erfasst, wobei die EMG die Muskelaktionspotentiale mittels differenzieller Spannungsmessung misst. Diese Signale können entweder invasiv mittels Nadelelektroden oder nichtinvasiv mittels Oberflächenelektroden abgeleitet werden. Die in den Signalen enthaltenen Informationen werden häufig zur Ansteuerung von Prothesen oder Myoprothesen genutzt, ermöglichen jedoch auch Rückschlüsse auf den Gesundheitszustand eines Muskels oder eine Muskelgruppe.

Ein Problem der Elektromyographie ist jedoch, dass die Nutzsignale einen stochastischen Charakter haben und typischerweise im niedrigen Frequenzbereich (<1000 Hz) liegen, in dem sie häufig von Störungen überlagert werden. Dadurch wird die Separation zwischen Nutz- und Störsignalen erschwert. Zudem kann durch die Applizierung von Oberflächenelektroden nicht gezielt ein bestimmter Muskel vermessen werden. Die geometrischen Überlagerungen mehrerer Muskeln im betrachteten Messbereich führen auch zu einer Unsicherheit bezüglich des physiologischen Ursprungs eines Signals.

Es ist jedoch essentiell, dass Prothesen sicher angesteuert werden. Bisherige Ansätze zur Unterscheidung, ob es sich bei dem aufgezeichneten Signal tatsächlich um ein EMG handelt oder um Störspannungen haben jedoch unterschiedliche Nachteile oder Einschränkungen. Bisherige Methoden zur Optimierung von Muskelkontraktions-Erkennungen haben in der Vergangenheit unterschiedliche Ansätze verfolgt. So wurde der Informationsgehalt von Messungen durch Hinzufügen von Redundanzen mittels Sensorfusionen erhöht. Bei den zusätzlichen Messungen am selben Messort handelt es sich typischerweise um die Mechanomyographie (MMG) oder die elektrische Impedanzmyographie (EIM). Diese Verfahren reagieren jedoch ähnlich dem EMG auf Bewegungsartefakte und sind in derzeit bekannten Umsetzungen nicht in der Lage, die Kontraktionen unterschiedlicher übereinanderliegender Muskeln voneinander zu unterscheiden.

Auch die Verwendung mehrerer EMG-Kanäle findet bereits häufig Anwendung, insbesondere in Form von EMG-Arrays, deren Platzierung mehrere Muskelgruppen abdecken kann. Bisherige Ansätze vermessen jedoch ausschließlich die Signalstärken zwischen den Elektroden. Diese werden häufig in Form von Heatmaps in 2-dimensionaler Darstellung ausgewertet. Es entstehen somit unter guten Messbedingungen Bilddaten, in denen der geometrische Verlauf eines einzelnen kontrahierten Muskels abgebildet wird.

Ein wesentlicher Nachteil der bisherigen Methoden ist, dass lediglich die Messbereiche ohne Muskelüberlagerungen eindeutig der Kontraktion eines Muskels zugeordnet werden können. In Bereichen von Überlagerungen, wie sie häufig vorkommen, kommen mehrere Muskeln als Signalquelle in Betracht. Somit bietet die erheblich komplexere EMG-Messung mittels Arrays nur einen relativ geringen Anteil an nützlichen Mehrinformationen.

Es ist daher die Aufgabe der vorliegenden Erfindung, die Nachteile aus dem bekannten Stand der Technik zu überwinden, insbesondere ein Verfahren bereitzustellen, bei dem die Robustheit von EMG-Messungen erhöht werden kann und der physiologische Ursprungs eines gemessenen EMG-Signals unterschieden werden kann. Eine weitere Aufgabe der vorliegenden Erfindung ist es, Störungen von tatsächlichen Muskelkontraktionen besser unterscheidbar zu machen und detailliertere Informationen bei EMG-Messungen bereitstellen zu können.

Diese Aufgaben werden durch die Gegenstände der unabhängigen Ansprüche gelöst.

KURZE BESCHREIBUNG DER ERFINDUNG

Die Hauptaspekte der vorliegenden Erfindung können beispielhaft wie folgt beschrieben werden:

In einem ersten Aspekt betrifft die vorliegende Erfindung ein Verfahren zur Separation von Nutz- und Störsignalen in einer elektromyografischen (EMG)-Messung und/oder zur Bestimmung des Signal-verursachenden Muskels in einer elektrisch aktiven Region an Muskeln.

In einem zweiten Aspekt betrifft die vorliegende Erfindung eine Verwendung eines Verfahrens nach dem ersten Aspekt der vorliegenden Erfindung zur EMG-Diagnostik von mindestens einem Muskel, insbesondere zur EMG-Diagnostik von mindestens zwei überlagernden Muskeln, zur Ansteuerung von Prothesen, zum Training von Muskeln, insbesondere zum Reha-Training und/oder zum Fitness-Training, oder zur Steuerung von Computer-Programmen und/oder Computer-Spielen.

In einem dritten Aspekt betrifft die vorliegende Erfindung ein computerimplementiertes Verfahren.

In einem vierten Aspekt betrifft die vorliegende Erfindung eine Vorrichtung zur Datenverarbeitung, umfassend Mittel zur Ausführung des Verfahrens nach dem dritten Aspekt der Erfindung.

In einem fünften Aspekt betrifft die vorliegende Erfindung ein Computerprogrammprodukt, umfassend Befehle, die bei der Ausführung des Programms durch einen Computer diesen veranlassen, das Verfahren nach dem dritten Aspekt der Erfindung auszuführen.

In einem sechsten Aspekt betrifft die vorliegende Erfindung ein computerlesbares Speichermedium, umfassend Befehle, die bei der Ausführung durch einen Computer diesen veranlassen, das Verfahren nach dem dritten Aspekt der Erfindung auszuführen.

In einem siebten Aspekt betrifft die vorliegende Erfindung eine Vorrichtung zur Separation von Nutz- und Störsignalen in einer elektromyografischen (EMG)-Messung und/oder zur Bestimmung des Signal-verursachenden Muskels in einer elektrisch aktiven Region an Muskeln.

DETAILLIERTE BESCHREIBUNG DER ERFINDUNG

Im Folgenden werden die Elemente der Erfindung beschrieben. Diese Elemente sind mit spezifischen Ausführungsformen aufgeführt, wobei diese in beliebiger Weise und in beliebiger Anzahl kombiniert werden können, um zusätzliche Ausführungsformen zu schaffen. Die unterschiedlich beschriebenen Beispiele und bevorzugten Ausführungsformen sollten nicht so ausgelegt werden, dass sie die vorliegende Erfindung nur auf die explizit beschriebenen Ausführungsformen beschränken. Diese Beschreibung der Erfindung sollte so verstanden werden, dass sie Ausführungsformen unterstützt und umfasst, die zwei oder mehr der explizit beschriebenen Ausführungsformen kombinieren oder die eine oder mehrere der explizit beschriebenen Ausführungsformen mit einer beliebigen Anzahl der offenbarten und/oder bevorzugten Elemente kombinieren. Darüber hinaus sollten alle Permutationen und Kombinationen aller beschriebenen Elemente in dieser Anmeldung als durch die Beschreibung der vorliegenden Anmeldung offenbart betrachtet werden, sofern sich aus dem Kontext nichts anderes ergibt.

Die vorstehende Aufgabe wird in einem ersten Aspekt gelöst, indem ein Verfahren zur Separation von Nutz- und Störsignalen in einer elektromyografischen (EMG)-Messung und/oder zur Bestimmung des Signal-verursachenden Muskels in einer elektrisch aktiven Region an Muskeln bereitgestellt wird, das Verfahren die folgenden Schritte umfassend:

(a) Anbringen von mindestens drei, vorzugsweise vier, fünf oder mehr, Elektroden auf unterschiedlichen Positionen auf mindestens zwei unterschiedlichen Achsen auf der Hautoberfläche und/oder in mindestens einem Muskel eines Subjekts, wobei die mindestens zwei unterschiedlichen Achsen eine mindestens erste und eine mindestens zweite Achse sind;

(b) Detektieren von mindestens einem EMG-Signal, das die myoelektrische Aktivität einer elektrisch aktiven Region an Muskeln des Subjekts beschreibt, mit den mindestens drei Elektroden;

(c) Messen der Spannung entlang der mindestens ersten und der mindestens zweiten Achse, und

(d) Bestimmen einer muskulären Ausbreitungsrichtung des mindestens einem EMG-Signals.

Es wurde herausgefunden, dass das Bestimmen einer muskulären Ausbreitungsrichtung des mindestens einem EMG-Signals vorteilhaft für die Separation von Nutz- und Störsignalen in der EMG-Messung und/oder zur Bestimmung des Signal-verursachenden Muskels in einer elektrisch aktiven Region an Muskeln ist. Die erfindungsgemäße Lösung des zugrundeliegenden Problems ermöglicht somit die Erkennung des Signal-verursachenden Muskels in einer EMG-Messung, eine bessere Unterscheidbarkeit von Störungen und tatsächlichen Muskelkontraktionen bei EMG-Messungen und eine Erhöhung der Robustheit von EMG-Messungen. Die zusätzliche Bestimmung der Ausbreitungsrichtung des gemessenen Signals gibt Rückschlüsse auf die Plausibilität eines Signals und erhöht somit die Störsicherheit.

Eine elektromyografische (EMG)-Messung im Sinne dieser Erfindung bezeichnet die Messung von Muskelaktionspotentialen mittels differenzieller Spannungsmessung.

Der Begriff "mindestens ein" soll im Sinne dieser Erfindung als zumindest ein einziger Gegenstand oder ein einziges Signal, oder 2, 3, 4, 5, 6, 7, 8, 9, 10, 11, 12, 13, 14, 15, 16, 17, 18, 19, 20, oder jede beliebige Anzahl an Gegenständen oder Signalen verstanden werden. So soll beispielsweise der Begriff "mindestens ein EMG-Signal" im Sinne dieser Erfindung als zumindest ein EMG-Signal, oder 2, 3, 4, 5, 6, 7, 8, 9, 10, 11, 12, 13, 14, 15, 16, 17, 18, 19, 20, oder jede beliebige Anzahl an EMG-Signalen verstanden werden. Weiterhin soll der Begriff "mindestens drei" Elektroden im Sinne dieser Erfindung als zumindest drei, oder vier, fünf, sechs, oder jede beliebige Anzahl an Elektroden verstanden werden.

Für die vorliegende Erfindung sollen mindestens drei Elektroden verwendet werden, denn so können mindestens zwei Differenzsignale in unterschiedlichen Richtungen gemessen werden. Das mindestens eine EMG-Signal, welches im Schritt b) des Verfahrens detektiert wird, wird aus den beiden Signalkomponenten, also den zwei differentiellen Spannungsmessungen, die in unterschiedlichen Richtungen gemessen werden, bestimmt.

Im Kontext dieser Erfindung bezieht sich der Begriff "Subjekt" vorzugsweise auf ein Säugetier, wie z.B. eine Maus, eine Ratte, ein Meerschweinchen, ein Kaninchen, eine Katze, ein Hund, ein Affe oder vorzugsweise einen Menschen, z.B. einen menschlichen Patienten. Das Subjekt kann an einer Krankheit leiden oder dem Risiko ausgesetzt sein, an einer Krankheit zu leiden.

Im Sinne dieser Erfindung bezeichnet der Schritt c) des Verfahrens das Ableiten der Spannung entlang der mindestens ersten und der mindestens zweiten Achse, welches vorzugsweise ein zeitkontinuierliches Ableiten der differentiellen Spannungen in den mindestens zwei Achsen ist. Unter der Formulierung "eine mindestens erste Achse" ist insbesondere "mindestens eine erste Achse" oder "eine erste Achse" zu verstehen. Unter der Formulierung "eine mindestens zweite Achse" ist insbesondere "mindestens eine zweite Achse" oder "eine zweite Achse" zu verstehen. Vorzugsweise werden die zuvor und nachfolgend beschriebenen Schritte gemäß ihrer alphabetischen, beispielsweise Schritt (b) nach Schritt (a), oder ihrer numerischen, beispielsweise Schritt (ii) nach Schritt (i), Reihenfolge nacheinander durchgeführt. Soweit möglich kann aber auch eine umgekehrte Reihenfolge, beispielsweise Schritt (d) vor Schritt (c), oder eine gleichzeitige Durchführung mehrere Schritte erfolgen.

In einer beispielhaften Weiterbildung des erfindungsgemäßen Verfahrens, umfasst das Verfahren zusätzlich den Schritt:
(e) Bereinigen des detektierten mindestens einem EMG-Signals.

In einer weiteren bevorzugten Ausführungsform umfasst das Verfahren zusätzlich den Schritt:
(f) Visualisieren und/oder Anzeigen der muskulären Ausbreitungsrichtung des mindestens einem EMG-Signals als mindestens ein Vektor.

Das erfindungsgemäße Verfahren ist nützlich für das Visualisieren und/oder Anzeigen der muskulären Ausbreitungsrichtung des mindestens einem EMG-Signals, woraus beispielsweise abgeleitet werden kann, welcher der Signal-verursachende Muskel in einer elektrisch aktiven Region an Muskeln ist.

In einer beispielhaften Weiterbildung des erfindungsgemäßen Verfahrens wird die muskuläre Ausbreitungsrichtung des mindestens einem EMG-Signals in Schritt (d) des Verfahrens bestimmt durch:

(i) Demodulation des detektierten mindestens einem EMG-Signals durch Anwenden einer Mittelungsfunktion auf mindestens eine Signalamplitude der Spannung entlang der mindestens ersten Achse und mindestens eine Signalamplitude der Spannung entlang der mindestens zweiten Achse und dadurch Erzeugen einer gemittelten Signalamplitude der Spannung entlang der mindestens ersten Achse und einer gemittelten Signalamplitude der Spannung entlang der mindestens zweiten Achse, und
(ii) Bestimmen der muskulären Ausbreitungsrichtung des Nutzsignals durch einen Abgleich der gemittelten Signalamplituden.

In Schritt (i) der zuvor beschriebenen beispielhaften Weiterbildung wird eine Mittelungsfunktion auf die detektierten EMG-Signalamplituden, also die ursprünglich detektierten, rohen und unbearbeiteten EMG-Signale angewandt, wodurch gemittelte EMG-Signalamplituden, also gemittelte Signalleistungen, beispielsweise durch Berechnung der Einhüllenden erzeugt werden. Wie es dem Fachmann geläufig ist, ist die Mittelungsfunktion in (i) eine Möglichkeit eine erfindungsgemäße Demodulation der Signale durchzuführen. In Schritt (ii) der zuvor beschriebenen beispielhaften Weiterbildung wird dann aus den demodulierten Signalen die muskuläre Ausbreitungsrichtung bestimmt.

In einer anderen beispielhaften Weiterbildung des erfindungsgemäßen Verfahrens wird die muskuläre Ausbreitungsrichtung des mindestens einem EMG-Signals in Schritt (d) des Verfahrens bestimmt durch:

(i) Extrahieren der Morphologie mindestens eines Signalabschnitts der Spannung entlang der mindestens ersten Achse und mindestens eines Signalabschnitts der Spannung entlang der mindestens zweiten Achse;
(ii) Vergleichen der Morphologie des mindestens einen Signalabschnitts der Spannung entlang der mindestens ersten Achse und des mindestens einen Signalabschnitts der Spannung entlang der mindestens zweiten Achse;
(iii) Auslegen und/oder Definieren mindestens eines Anteils des Signalabschnitts entlang der mindestens ersten Achse und eines Anteils des Signalabschnitts entlang der mindestens zweiten Achse, die einen Anteil an einer Ähnlichkeit aufweisen, als ein Nutzsignal, und
(iv) Bestimmen der muskulären Ausbreitungsrichtung des Nutzsignals mittels Demodulation.

In der zuvor beschriebenen beispielhaften Weiterbildung werden die Rohsignale mindestens eines Signalabschnitts der Spannung entlang der mindestens ersten Achse und mindestens eines Signalabschnitts der Spannung entlang der mindestens zweiten Achse, also deren Morphologien miteinander verglichen. Die Signalanteile, die zueinander ähnlich sind, werden dann als Nutzsignale interpretiert und für die Demodulation herangezogen.

Durch unterschiedliche Verfahren der Signalverarbeitung werden die sich ähnelnden Signalanteile für die weiteren Schritte extrahiert und die Unterschiede entfernt. Derartige Verfahren sind dem Fachmann geläufig. Beispielsweise können mittels Frequenzfilter Spektralanteile entfernt werden, die besonders unterschiedlich sind.

Weiterhin kann der Anteil an der Ähnlichkeit erfindungsgemäß bestimmt werden durch ein Verfahren umfassend:

(i) Faltung;

(ii) Kreuzkorrelation;

(iii) Filterung;

(iv) Signalsubtraktion;

(v) Eine lineare mathematische Operation;

(vi) Eine nicht-lineare mathematische Operation;

(vii) Multiplikation;

(viii) Division;

(ix) Wavelet Transformation;

(x) Empirical Mode Decomposition;

(xi) Entfernen von unterschiedlichen Spektralanteilen mittels mindestens eines Frequenzfilters; und/oder

(xii) Mindestens ein Algorithmus, beispielsweise ein lernender Algorithmus.

In einer bevorzugten Ausführungsform wird die muskuläre Ausbreitungsrichtung des mindestens einem EMG-Signals, und/oder der Vektor, mit mindestens einer Referenzmorphologie von einem Gewebe und/oder einer Muskellage verglichen, wobei dadurch die Bestimmung des Signal-verursachenden Muskels in einer elektrisch aktiven Region an Muskeln und/oder dadurch ein automatisiertes Erlernen der Platzierung der mindestens drei Elektroden ermöglicht wird.

In einer beispielhaften Weiterbildung des erfindungsgemäßen Verfahrens werden die mindestens drei Elektroden auf unterschiedlichen Positionen oberhalb von mindestens zwei übereinanderliegenden Muskeln auf der Hautoberfläche eines Subjekts in Schritt a) des Verfahrens angebracht.

Erfindungsgemäß kann bei dem allgemeinen Fall mit mehreren Muskeln entsprechend des Verfahrens für jeden Muskel einzeln eine muskuläre Ausbreitungsrichtung mittels der 2-kanaligen differentiellen Spannungsmessung erfindungsgemäß bestimmt werden.

Sofern die Elektroden auf orthogonalen Achsen positioniert sind, können die beiden Signalamplituden unmittelbar in einen Betrag und in eine Phase des Signalvektors umgerechnet werden:

$$|EMG| = \sqrt{Kanal1^2 + Kanal2^2}|$$

bzw.

$$\varphi = arctan2(Kanal1, Kanal2)$$

Legt man den hierdurch berechneten Vektor gemäß des von den Elektroden aufgespannten Koordinatensystems auf die Elektrodenanordnung, so zeigt dieser die muskuläre Ausbreitungsrichtung des EMG-Signals, welche mit der Muskelfaserrichtung des darunterliegenden, angespannten Muskels übereinstimmt.

Eine andere beispielhafte Weiterbildung des erfindungsgemäßen Verfahrens betrifft dann das Verfahren, wobei das Detektieren des mindestens einem EMG-Signals mit den mindestens drei Elektroden in Schritt (b) des Verfahrens an einer Einzelposition oder an unterschiedlichen Positionen erfolgt.

Wie dem Fachmann geläufig ist, wird ein EMG alle "in der Nähe" liegenden Muskeln erfassen. In einer Ausführungsform sollten die Elektroden vorzugsweise geometrisch oberhalb des betrachteten Muskels angebracht werden. In einer beispielhaften Weiterbildung kann das Detektieren des EMG-Signals mit den mindestens drei Elektroden in Schritt (b) des Verfahrens an einer Messposition im Zentrum zwischen den Elektroden erfolgen.

In einer weiteren beispielhaften Weiterbildung des erfindungsgemäßen Verfahrens sind die mindestens drei Elektroden ein Elektrodenarray.

In einer anderen bevorzugten Ausführungsform des Verfahrens wird die muskuläre Ausbreitungsrichtung des mindestens einem EMG-Signals zur Erkennung des Signal-verursachenden und/oder kontrahierten Muskels genutzt.

In einer weiteren beispielhaften Weiterbildung des erfindungsgemäßen Verfahrens sind die Elektroden Oberflächenelektroden und/oder Nadelelektroden, wobei die Elektroden vorzugsweise Oberflächenelektroden sind.

In einer anderen beispielhaften Weiterbildung des erfindungsgemäßen Verfahrens sind die Elektroden äquidistante Elektroden und/oder die Elektroden sind auf orthogonalen Achsen positioniert, wobei orthogonal im Sinne dieser Erfindung einen Winkel von etwa 90° bezeichnet. Im Sinne dieser Erfindung wird der Begriff "etwa" verwendet, um eine gewisse Mengentoleranz anzudeuten. "Etwa" bezeichnet erfindungsgemäß $\pm$ 10.0%, oder $\pm$ 5.0%, oder $\pm$ 1.0%, oder $\pm$ 0.5%, oder $\pm$ 0.1%.

In einer weiteren bevorzugten Ausführungsform ist das Verfahren ein nichtinvasives Verfahren.

In einer beispielhaften Weiterbildung des erfindungsgemäßen Verfahrens wird auf der gegenüberliegenden Seite der in Schritt (a) des Verfahrens angebrachten mindestens drei Elektroden eine Referenzelektrode zum schaltungstechnischen Potentialausgleich angebracht.

In einer weiteren bevorzugten Ausführungsform sind die mindestens drei Elektroden mit mindestens zwei EMG-Kanälen, oder drei, oder vier, oder mehr EMG-Kanälen verbunden.

In einer beispielhaften Weiterbildung des erfindungsgemäßen Verfahrens ist die detektierte EMG-Signalamplitude in einem EMG-Kanal hoch, der in Muskelfaserrichtung eines kontrahierenden Muskels misst, und/oder die EMG-Signalamplitude in einem EMG-Kanal niedrig, der orthogonal der Muskelfaserrichtung eines kontrahierenden Muskels misst.

Beispielsweise kann die detektierte EMG-Signalamplitude in einem ersten EMG-Kanal und in einem zweiten EMG-Kanal in etwa gleich groß sein, wenn mehrere Signal-verursachende Muskeln in einer elektrisch aktiven Region an Muskeln gleichzeitig in etwa gleicher Stärke kontrahieren. Weiterhin kann die detektierte EMG-Signalamplitude in einem ersten EMG-Kanal und in einem zweiten EMG-Kanal ungleich sein, wenn einer oder ein Teil mehrerer Signal-verursachenden Muskeln in einer elektrisch aktiven Region an Muskeln gleichzeitig kontrahieren, und/oder mehrere Signal-verursachende Muskeln in einer elektrisch aktiven Region an Muskeln gleichzeitig in ungleicher Stärke kontrahieren.

In einer weiteren bevorzugten Ausführungsform umfasst das Verfahren zusätzlich die Schritte:

> (g) Messen einer elektrischen Bioimpedanz und/oder einer Impedanzmyographie (EIM), und
> (h) Vergleichen des mindestens einem EMG-Signals mit der geometrischen Verteilung und/oder der zeitlichen Änderung der elektrischen Bioimpedanz und/oder der Impedanzmyographie (EIM).

In einer beispielhaften Weiterbildung des erfindungsgemäßen Verfahrens umfasst das Verfahren zusätzlich den Schritt:
(i) Extrahieren eines Mechanomyogramms (MMG)-Signals von einem oder mehreren Vibrationssensoren als Reaktion auf eine Oberflächenvibration eines Muskels des Subjekts.

In einer anderen bevorzugten Ausführungsform umfasst das Verfahren zusätzlich den Schritt:
(j) Rückmelden an das Subjekt oder den Benutzer, welcher Muskel kontrahiert ist.

In einer beispielhaften Weiterbildung des erfindungsgemäßen Verfahrens umfasst das Verfahren zusätzlich den Schritt:
(k) Überwachen, insbesondere digitales Überwachen, eines Trainings von mindestens einem Muskel.

In einer bevorzugten Ausführungsform umfasst das Verfahren zusätzlich den Schritt:
(l) Erkennen und/oder Unterdrücken von Störsignalen von mindestens einem anderen Muskel in der elektrisch aktiven Region an Muskeln, beispielsweise dem Signal des Herzmuskels.

Ein konkretes Anwendungsbeispiel des erfindungsgemäßen Verfahrens liegt in der Verwendung des Verfahrens in der EMG-Diagnostik, insbesondere der EMG-Diagnostik von mindestens zwei überlagernden Muskeln, der Ansteuerung von Prothesen, dem Training von Muskeln, insbesondere dem Reha-Training und/oder dem Fitness-Training, oder der Steuerung von Computer-Programmen und/oder Computer-Spielen.

Gemäß einem zweiten Aspekt der vorliegenden Erfindung, der mit den vorhergehenden Aspekten und beispielhaften Ausführungen kombinierbar ist, wird die vorstehende Aufgabe also gelöst, indem eine Verwendung eines Verfahrens nach dem ersten Aspekt der vorliegenden Erfindung zur EMG-Diagnostik von mindestens einem Muskel, insbesondere zur EMG-Diagnostik von mindestens zwei überlagernden Muskeln, zur Ansteuerung von Prothesen, zum Training von Muskeln, insbesondere zum Reha-Training und/oder zum Fitness-Training, oder zur Steuerung von Computer-Programmen und/oder Computer-Spielen bereitgestellt wird.

Ein weiteres Anwendungsbeispiel des erfindungsgemäßen Verfahrens liegt zudem in einer medizinischen Verwendung. Eine medizinische "Verwendung" im Rahmen dieser Erfindung bezieht sich vorzugsweise auf ein Verfahren zur Vorbeugung oder Behandlung einer Krankheit in einem Subjekt, wobei das Verfahren einen Schritt umfasst, in welchem dem Subjekt eine therapeutisch wirksame Menge eines therapeutisch wirksamen Stoffes verabreicht wird.

Unter Behandlung versteht man z.B. die Behandlung, Verzögerung oder Linderung des Krankheitsverlaufs, die Verringerung der Symptome oder die Heilung der Krankheit oder des Leidens. Eine "effektive Menge" ist eine Menge, welche Symptome lindert, die für die zu behandelnde Krankheit, wie z.B. eine degenerative und/oder eine Hauterkrankung, festgestellt wurden. Unter Linderung versteht man z.B. das Vorbeugen, Behandeln und Reduzieren der Symptome oder das Heilen der Krankheit oder des Leidens. Die Erfindung beinhaltet auch ein Verfahren zur Behandlung eines gefährdeten Subjekts für die Entwicklung und/oder das Fortschreiten einer Erkrankung, worin dem Subjekt oder Patienten eine therapeutisch wirksame Menge verabreicht wird.

Gemäß einem dritten Aspekt der vorliegenden Erfindung, der mit den vorhergehenden Aspekten und beispielhaften Ausführungen kombinierbar ist, wird die vorstehende Aufgabe gelöst, indem ein computerimplementiertes Verfahren bereitgestellt wird, umfassend den Schritt:

> (a) Bestimmen einer muskulären Ausbreitungsrichtung mindestens eines EMG-Signals.

Erfindungsgemäß können die zuvor nach dem ersten Aspekt der Erfindung gemessenen EMG-Signale in das computerimplementierte Verfahren gemäß dem dritten Aspekt der vorliegenden Erfindung eingespeist werden.

Weiterhin betrifft die vorliegende Erfindung auch ein computerimplementiertes Verfahren, umfassend die Schritte:

> (a) Detektieren eines EMG-Signals, das die myoelektrische Aktivität einer elektrisch aktiven Region an Muskeln des Subjekts beschreibt, mit den mindestens drei Elektroden, und/oder
> (b) Bestimmen einer Signalamplitude und einer Ausbreitungsrichtung des EMG-Signals.

Ein weiterer Aspekt der vorliegenden Erfindung, der mit den vorhergehenden Aspekten und beispielhaften Ausführungen kombinierbar ist, bezieht sich dann auf eine Vorrichtung oder ein System zur Datenverarbeitung, umfassend Mittel zur Ausführung des Schritts (a) des Verfahrens nach dem dritten Aspekt der Erfindung.

Diese Erfindung umfasst auch eine Vorrichtung oder ein System zur Datenverarbeitung, umfassend Mittel zur Ausführung des Schritts

a) Detektieren eines EMG-Signals, das die myoelektrische Aktivität einer elektrisch aktiven Region an Muskeln des Subjekts beschreibt, mit den mindestens drei Elektroden, und/oder

b) Bestimmen einer Signalamplitude und einer Ausbreitungsrichtung des EMG-Signals.

Gemäß einem fünften Aspekt der vorliegenden Erfindung, der mit den vorhergehenden Aspekten und beispielhaften Ausführungen kombinierbar ist, wird die vorstehende Aufgabe gelöst, indem ein Computerprogrammprodukt bereitgestellt wird, umfassend Befehle, die bei der Ausführung des Programms durch einen Computer diesen veranlassen, den Schritt (a) des Verfahrens nach dem dritten Aspekt der Erfindung auszuführen.

Darüber hinaus umfasst diese Erfindung auch ein Computerprogrammprodukt, umfassend Befehle, die bei der Ausführung des Programms durch einen Computer diesen veranlassen, die Schritte

a) Detektieren eines EMG-Signals, das die myoelektrische Aktivität einer elektrisch aktiven Region an Muskeln des Subjekts beschreibt, mit den mindestens drei Elektroden, und/oder

b) Bestimmen einer Signalamplitude und einer Ausbreitungsrichtung des EMG-Signals auszuführen.

Ein weiterer Aspekt dieser Erfindung, der mit den vorhergehenden Aspekten und beispielhaften Ausführungen kombinierbar ist, bezieht sich auf ein computerlesbares Speichermedium, umfassend Befehle, die bei der Ausführung durch einen Computer diesen veranlassen, den Schritt (a) des Verfahrens nach dem dritten Aspekt der Erfindung auszuführen.

Diese Erfindung umfasst auch ein computerlesbares (Speicher)medium, umfassend Befehle, die bei der Ausführung durch einen Computer diesen veranlassen, die Schritte

a) Detektieren eines EMG-Signals, das die myoelektrische Aktivität einer elektrisch aktiven Region an Muskeln des Subjekts beschreibt, mit den mindestens drei Elektroden, und/oder

b) Bestimmen einer Signalamplitude und einer Ausbreitungsrichtung des EMG-Signals auszuführen.

Gemäß einem siebten Aspekt der vorliegenden Erfindung, der mit den vorhergehenden Aspekten und beispielhaften Ausführungen kombinierbar ist, wird die vorstehende Aufgabe in einem weiteren Aspekt gelöst, indem eine Vorrichtung zur Separation von Nutz- und Störsignalen in einer elektromyografischen (EMG)-Messung und/oder zur Bestimmung des Signal-verursachenden Muskels in einer elektrisch aktiven Region an Muskeln bereitgestellt wird, umfassend:

(a) Mindestens drei, vorzugsweise vier, fünf oder mehr, Elektroden;
(b) Mindestens zwei, vorzugsweise drei, vier, fünf oder mehr, EMG-Kanäle, und
(c) Mindestens eine Einheit zum Detektieren von mindestens einem EMG-Signal, das die myoelektrische Aktivität einer elektrisch aktiven Region an Muskeln des Subjekts beschreibt.

Die vorliegende Erfindung wird anhand der folgenden Gegenstände beschrieben, welche bevorzugte Ausführungsformen der Erfindung darstellen.

Gegenstand 1: Ein Verfahren zur Separation von Nutz- und Störsignalen in einer elektromyografischen (EMG)-Messung und/oder zur Bestimmung des Signal-verursachenden Muskels in einer elektrisch aktiven Region an Muskeln, die folgenden Schritte umfassend:

(a) Anbringen von mindestens drei, vorzugsweise vier, fünf oder mehr, Elektroden auf unterschiedlichen Positionen auf mindestens zwei unterschiedlichen Achsen auf der Hautoberfläche und/oder in mindestens einem Muskel eines Subjekts, wobei die mindestens zwei unterschiedlichen Achsen eine mindestens erste und eine mindestens zweite Achse sind;
(b) Detektieren von mindestens einem EMG-Signal, das die myoelektrische Aktivität einer elektrisch aktiven Region an Muskeln des Subjekts beschreibt, mit den mindestens drei Elektroden;
(c) Messen der Spannung entlang der mindestens ersten und der mindestens zweiten Achse, und
(d) Bestimmen einer muskulären Ausbreitungsrichtung des mindestens einem EMG-Signals.

Gegenstand 2: Das Verfahren nach Gegenstand 1, wobei das Verfahren zusätzlich den Schritt umfasst:
(e) Bereinigen des detektierten mindestens einem EMG-Signals.

Gegenstand 3: Das Verfahren nach Gegenstand 1 oder 2, wobei das Verfahren zusätzlich den Schritt umfasst:
(f) Visualisieren und/oder Anzeigen der muskulären Ausbreitungsrichtung des mindestens einem EMG-Signals als mindestens ein Vektor.

Gegenstand 4: Das Verfahren nach einem der Gegenstände 1 bis 3, wobei die muskuläre Ausbreitungsrichtung des mindestens einem EMG-Signals in Schritt (d) des Verfahrens bestimmt wird durch:

(i) Demodulation des detektierten mindestens einem EMG-Signals durch Anwenden einer Mittelungsfunktion auf mindestens eine Signalamplitude der Spannung entlang der mindestens ersten Achse und mindestens eine Signalamplitude der Spannung entlang der mindestens zweiten Achse und dadurch Erzeugen einer gemittelten Signalamplitude der Spannung entlang der mindestens ersten Achse und einer gemittelten Signalamplitude der Spannung entlang der mindestens zweiten Achse, und

(ii) Bestimmen der muskulären Ausbreitungsrichtung des Nutzsignals durch einen Abgleich der gemittelten Signalamplituden.

Gegenstand 5: Das Verfahren nach einem der Gegenstände 1 bis 3, wobei die muskuläre Ausbreitungsrichtung des mindestens einem EMG-Signals in Schritt (d) des Verfahrens bestimmt wird durch:

(i) Extrahieren der Morphologie mindestens eines Signalabschnitts der Spannung entlang der mindestens ersten Achse und mindestens eines Signalabschnitts der Spannung entlang der mindestens zweiten Achse;

(ii) Vergleichen der Morphologie des mindestens einen Signalabschnitts der Spannung entlang der mindestens ersten Achse und des mindestens einen Signalabschnitts der Spannung entlang der mindestens zweiten Achse;

(iii) Auslegen und/oder Definieren mindestens eines Anteils des Signalabschnitts entlang der mindestens ersten Achse und eines Anteils des Signalabschnitts entlang der mindestens zweiten Achse, die einen Anteil an einer Ähnlichkeit aufweisen, als ein Nutzsignal, und

(iv) Bestimmen der muskulären Ausbreitungsrichtung des Nutzsignals mittels Demodulation.

Gegenstand 6: Das Verfahren nach Gegenstand 5, wobei der Anteil an der Ähnlichkeit bestimmt wird durch ein Verfahren umfassend:

(i) Faltung;
(ii) Kreuzkorrelation;
(iii) Filterung;
(iv) Signalsubtraktion;
(v) Eine lineare mathematische Operation;
(vi) Eine nicht-lineare mathematische Operation;
(vii) Multiplikation;
(viii) Division;
(ix) Wavelet Transformation;
(x) Empirical Mode Decomposition;
(xi) Entfernen von unterschiedlichen Spektralanteilen mittels mindestens eines Frequenzfilters; und/oder
(xii) Mindestens ein Algorithmus, beispielsweise ein lernender Algorithmus.

Gegenstand 7: Das Verfahren nach einem der Gegenstände 1 bis 6, wobei die muskuläre Ausbreitungsrichtung des mindestens einem EMG-Signals, und/oder der Vektor, mit mindestens einer Referenzmorphologie von einem Gewebe und/oder einer Muskellage verglichen wird, dadurch Bestimmung des Signal-verursachenden Muskels in einer elektrisch aktiven Region an Muskeln und/oder dadurch automatisiertes Erlernen der Platzierung der mindestens drei Elektroden.

Gegenstand 8: Das Verfahren nach einem der Gegenstände 1 bis 7, wobei die mindestens drei Elektroden auf unterschiedlichen Positionen oberhalb von mindestens zwei übereinanderliegenden Muskeln auf der Hautoberfläche eines Subjekts in Schritt a) des Verfahrens angebracht werden.

Gegenstand 9: Das Verfahren nach einem der Gegenstände 1 bis 8, wobei das Detektieren des mindestens einem EMG-Signals mit den mindestens drei Elektroden in Schritt (b) des Verfahrens an einer Einzelposition oder an unterschiedlichen Positionen erfolgt.

Gegenstand 10: Das Verfahren nach einem der Gegenstände 1 bis 9, wobei die mindestens drei Elektroden ein Elektrodenarray sind.

Gegenstand 11: Das Verfahren nach einem der Gegenstände 1 bis 10, wobei die muskuläre Ausbreitungsrichtung des mindestens einem EMG-Signals zur Erkennung des Signal-verursachenden und/oder kontrahierten Muskels genutzt wird.

Gegenstand 12: Das Verfahren nach einem der Gegenstände 1 bis 11, wobei die Elektroden Oberflächenelektroden und/oder Nadelelektroden sind, und wobei die Elektroden vorzugsweise Oberflächenelektroden sind.

Gegenstand 13: Das Verfahren nach einem der Gegenstände 1 bis 12, wobei die Elektroden äquidistante Elektroden sind und/oder die Elektroden auf orthogonalen Achsen positioniert sind.

Gegenstand 14: Das Verfahren nach einem der Gegenstände 1 bis 13, wobei das Verfahren ein nichtinvasives Verfahren ist.

Gegenstand 15: Das Verfahren nach einem der Gegenstände 1 bis 14, wobei auf der gegenüberliegenden Seite der in Schritt (a) des Verfahrens angebrachten mindestens drei Elektroden eine Referenzelektrode zum schaltungstechnischen Potentialausgleich angebracht wird.

Gegenstand 16: Das Verfahren nach einem der Gegenstände 1 bis 15, wobei die mindestens drei Elektroden mit mindestens zwei EMG-Kanälen verbunden sind.

Gegenstand 17: Das Verfahren nach Gegenstand 16, wobei die detektierte EMG-Signalamplitude in einem EMG-Kanal hoch ist, der in die Muskelfaserrichtung eines kontrahierenden Muskels misst, und/oder wobei die EMG-Signalamplitude in einem EMG-Kanal niedrig ist, der orthogonal der Muskelfaserrichtung eines kontrahierenden Muskels misst.

Gegenstand 18: Das Verfahren nach einem der Gegenstände 1 bis 17, wobei das Verfahren zusätzlich die Schritte umfasst:

(g) Messen einer elektrischen Bioimpedanz und/oder einer Impedanzmyographie (EIM), und
(h) Vergleichen des mindestens einem EMG-Signals mit der geometrischen Verteilung und/oder der zeitlichen Änderung der elektrischen Bioimpedanz und/oder der Impedanzmyographie (EIM).

Gegenstand 19: Das Verfahren nach einem der Gegenstände 1 bis 18, wobei das Verfahren zusätzlich den Schritt umfasst:
(i) Extrahieren eines Mechanomyogramms (MMG)-Signals von einem oder mehreren Vibrationssensoren als Reaktion auf eine Oberflächenvibration eines Muskels des Subjekts.

Gegenstand 20: Das Verfahren nach einem der Gegenstände 1 bis 19, wobei das Verfahren zusätzlich den Schritt umfasst:
(j) Rückmelden an das Subjekt oder den Benutzer, welcher Muskel kontrahiert ist.

Gegenstand 21: Das Verfahren nach einem der Gegenstände 1 bis 20, wobei das Verfahren zusätzlich den Schritt umfasst:
(k) Überwachen, insbesondere digitales Überwachen, eines Trainings von mindestens einem Muskel.

Gegenstand 22: Das Verfahren nach einem der Gegenstände 1 bis 21, wobei das Verfahren zusätzlich den Schritt umfasst:
(l) Erkennen und/oder Unterdrücken von Störsignalen von mindestens einem anderen Muskel in der elektrisch aktiven Region an Muskeln, beispielsweise dem Signal des Herzmuskels.

Gegenstand 23: Eine Verwendung eines Verfahrens nach einem der Gegenstände 1 bis 22 zur EMG-Diagnostik von mindestens einem Muskel, insbesondere zur EMG-Diagnostik von mindestens zwei überlagernden Muskeln, zur Ansteuerung von Prothesen, zum Training von Muskeln, insbesondere zum Reha-Training und/oder zum Fitness-Training, oder zur Steuerung von Computer-Programmen und/oder Computer-Spielen.

Gegenstand 24: Ein Computerimplementiertes Verfahren, umfassend den Schritt:

(a) Bestimmen einer muskulären Ausbreitungsrichtung mindestens eines EMG-Signals.

Gegenstand 25: Eine Vorrichtung zur Datenverarbeitung, umfassend Mittel zur Ausführung des Schritts (a) des Verfahrens nach Gegenstand 24.

Gegenstand 26: Ein Computerprogrammprodukt, umfassend Befehle, die bei der Ausführung des Programms durch einen Computer diesen veranlassen, den Schritt (a) des Verfahrens nach Gegenstand 24 auszuführen.

Gegenstand 27: Ein Computerlesbares Speichermedium, umfassend Befehle, die bei der Ausführung durch einen Computer diesen veranlassen, den Schritt (a) des Verfahrens nach Gegenstand 24 auszuführen.

Gegenstand 28: Eine Vorrichtung zur Separation von Nutz- und Störsignalen in einer elektromyografischen (EMG)-Messung und/oder zur Bestimmung des Signal-verursachenden Muskels in einer elektrisch aktiven Region an Muskeln, umfassend:

(a) Mindestens drei, vorzugsweise vier, fünf oder mehr, Elektroden;
(b) Mindestens zwei, vorzugsweise drei, vier, fünf oder mehr, EMG-Kanäle, und
(c) Mindestens eine Einheit zum Detektieren von mindestens einem EMG-Signal, das die myoelektrische Aktivität einer elektrisch aktiven Region an Muskeln des Subjekts beschreibt.

Bevorzugte Ausführungen der Erfindung sind in den Unteransprüchen angegeben.
Die hierin verwendeten Begriffe "nach der [vorliegenden] Erfindung", "erfindungsgemäß" und dergleichen sollen sich auf alle beschriebenen Aspekte und Ausführungsformen der Erfindung beziehen, die hierin beschrieben und/oder beansprucht werden.
Der Begriff "umfassend", wie er hierin verwendet wird, ist so auszulegen, dass er sowohl "einschließlich" als auch "bestehend aus" umfasst, wobei beide Bedeutungen spezifisch beabsichtigt sind und somit individuell offenbarte Ausführungsformen gemäß der vorliegenden Erfindung offenbart werden. Sofern hierin die Bezeichnung "und/oder" verwendet wird, ist die Bezeichnung "und/oder" als spezifische Offenbarung jedes der beiden spezifizierten Merkmale oder Komponenten mit oder ohne das andere zu verstehen. Zum Beispiel ist "A und/oder B" als spezifische Offenbarung von jedem von (i) A, (ii) B und (iii) A und B zu verstehen, so als ob jedes hier einzeln aufgeführt wäre. Im Zusammenhang mit der vorliegenden Erfindung bezeichnen die Begriffe "ungefähr" bzw. "etwa" ein Genauigkeitsintervall, welches der Fachmann versteht, um die technische Wirkung des betreffenden Merkmals dennoch sicherzustellen. Der Begriff gibt typischerweise eine Abweichung vom angegebenen Zahlenwert um ±20%, ±15%, ±10% und beispielsweise ±5% an. Wie der Durchschnittsfachmann erkennen wird, hängt die spezifische derartige Abweichung für einen numerischen Wert für einen gegebenen technischen Effekt von der Art des technischen Effekts ab. Beispielsweise kann ein natürlicher oder biologischer technischer Effekt im Allgemeinen eine größere solche Abweichung aufweisen als ein von Menschen verursachter Effekt. Sofern ein unbestimmter oder bestimmter Artikel verwendet wird, wenn man sich auf ein Nomen im Singular bezieht, z.B. "ein" oder "eine", so schließt dies den Plural dieses Nomens ein, es sei denn, es ist hierin etwas anderes ausdrücklich angegeben.

Sofern der Kontext nichts anderes vorschreibt, sind die Beschreibungen und Definitionen der oben dargelegten Merkmale nicht auf einen bestimmten Aspekt oder eine bestimmte Ausführungsform der Erfindung beschränkt und gelten gleichermaßen für alle beschriebenen Aspekte und Ausführungsformen der Erfindung.

KURZE BESCHREIBUNG DER FIGUREN

Im Folgenden werden weitere Eigenschaften, Merkmale und Vorteile der Erfindung mittels Beschreibung bevorzugter Ausführungen der Erfindung anhand der beiliegenden beispielhaften Zeichnungen und Beispiele näher beschrieben, ohne darauf beschränkt zu sein. Für die Zwecke der vorliegenden Erfindung werden alle hierin zitierten Referenzen in ihrer Gesamtheit aufgenommen.

In den Figuren ist Folgendes, wie nachfolgend beschrieben, gezeigt.

Figur 1 zeigt: (A) Eine exemplarische geometrische Überlagerung von Muskeln im Unterarm, in der zur vereinfachten Darstellung ausschließlich die Muskeln "abductor pollicis longus" (1) und "flexor carpi ulnaris" (2) eingezeichnet sind. (B) Zeigt einen exemplarischen Messansatz zur Bestimmung von Signalstärke und Ausbreitungsrichtung eines EMG-Signals in der in Figur 1 A gezeigten geometrische Anordnung von Muskeln im Unterarm.

Figur 2 zeigt ein EMG-Vektorfeld bei Kontraktion von Muskel 1 (A, "abductor pollicis longus") und Muskel 2 (B, "flexor carpi ulnaris") im Unterarm.

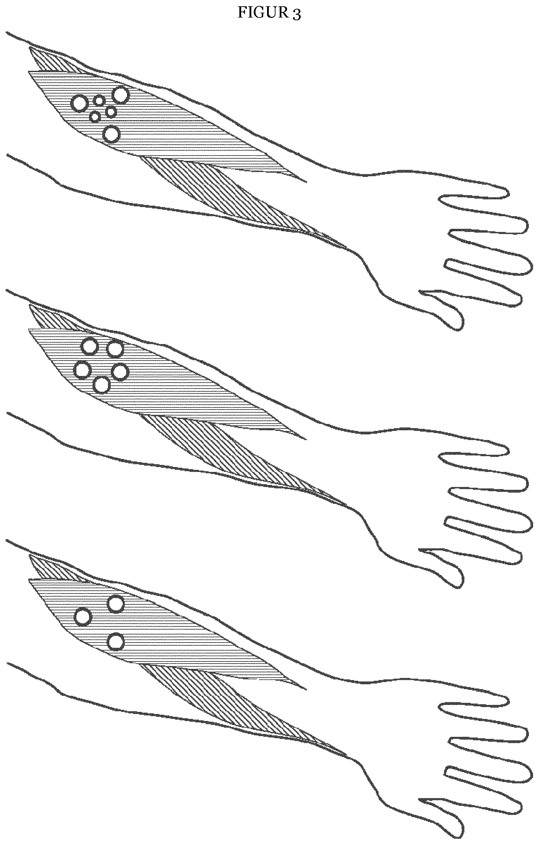

Figur 3 zeigt weitere erfindungsgemäße beispielhafte Elektrodenkonfigurationen in der in Figur 1A gezeigten geometrische Anordnung von Muskeln im Unterarm, durch deren Linearkombination eine Richtung bestimmt werden kann (3-Kanal, 5-Kanal und 6-Kanal Konfigurationen).

Figur 4 zeigt eine schematische Darstellung der Signalverarbeitung zur Bestimmung von Betrag und Phase bzw. Ausbreitungsrichtung des EMG-Signals.

Figur 5 zeigt: (A) Eine beispielhafte erfindungsgemäße Elektrodenkonfiguration, bei der eine orthogonale Anordnung zweier Kanäle mit je zwei äquidistanten Elektroden zur differentiellen erfindungsgemäßen Spannungsmessung genutzt wird. (B) Legt man den berechneten Vektor (schwarze, gestrichelte Linie) gemäß des von den Elektroden aufgespannten Koordinatensystems auf das Foto aus Figur 5A, so zeigt dieser die Ausbreitungsrichtung des EMG-Signals, welches mit der Muskelfaserrichtung des darunterliegenden, angespannten Muskels übereinstimmt.

Figur 6 zeigt eine Darstellung der zeitkontinuierlich abgeleiteten differentiellen Spannungen beider Kanäle aus Figur 5A für eine Dauer von 20 Sekunden. Abkürzungen: CH1 = Kanal 1; CH2 = Kanal 2.

Figur 7 zeigt eine Vergrößerung eines Zeitabschnitts aus Figur 6, wobei die Signale in (A) nicht normiert wurden, während die Signale in (B) vorab normiert wurden. Abkürzungen: CH1 = Kanal 1; CH2 = Kanal 2.

BEISPIELE

Unter Bezugnahme auf die Figuren werden Ausführungsbeispiele eines erfindungsgemäßen Verfahrens und dessen Verwendung beschrieben.
Die Beispiele zeigen:

**Beispiel 1: Verwertung der Ausbreitungsrichtung eines EMG-Signals**

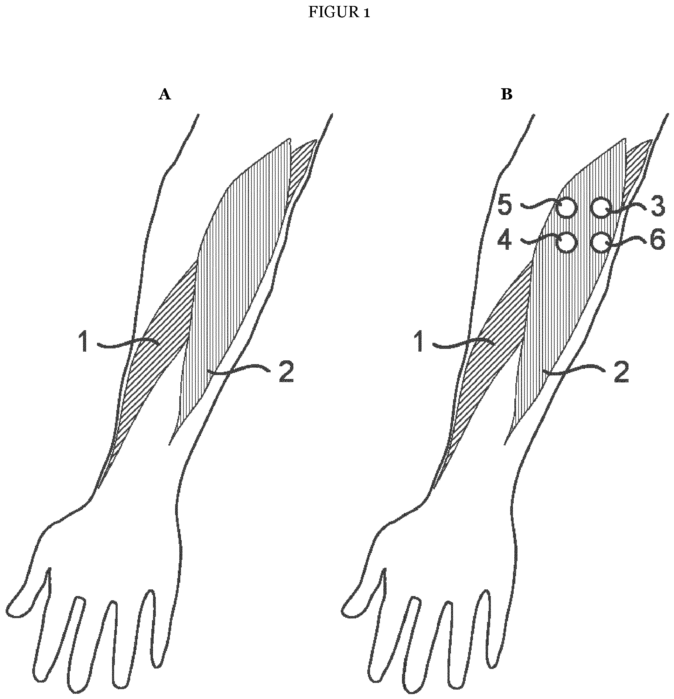

Im Gegensatz zu herkömmlichen Messmethoden wird in der vorliegenden Erfindung nicht nur der die Stärke eines EMG-Signals als Informationsquelle genutzt, sondern auch dessen Ausbreitungsrichtung. Dazu wird der betrachtete Messbereich von mindestens 2 unterschiedlich angeordneten Elektrodenpositionen betrachtet. Ein exemplarischer Messaufbau zur Bestimmung eines richtungsabhängigen EMG-Signals ist in Figur 1B gezeigt.
Die vier platzierten Elektroden (3, 4, 5, 6) zur Signalableitung befinden sich auf der Hautoberfläche oberhalb der beiden übereinanderliegenden Muskeln.

Die so angestrebte effektive Messposition befindet sich mittig zwischen den vier Elektroden. Die erste Möglichkeit, die Ausbreitungsrichtung des EMG-Signals zu bestimmen, ist die Bestimmung der gemittelten Signalstärken zwischen den einzelnen Elektroden und der anschließende Vergleich.

Im einfachsten Fall bedeutet dies eine differentielle Spannungsmessung zwischen 3 und 4 der in Figur 1B angedeuteten Elektroden, im folgenden "Kanal 1" genannt bzw. 5 und 6 der in Figur 1B angedeuteten Elektroden, im folgenden "Kanal 2" genannt. Ist der Muskel 1 kontrahiert, wird die Signalstärke in Kanal 1 sehr hoch sein, da dieser in Muskelfaserrichtung misst. Die von Kanal 2 aufgezeichnete Signalstärke wird hingegen sehr gering sein. Im Falle einer gleichzeitigen Muskelkontraktion von 1 und 2, teilt sich die gemessene Signalstärke auf beide Kanäle auf. Die so ermittelte Richtung des EMG-Signals wird anschließend zur Erkennung des kontrahierten Muskels genutzt. Unter Verwendung mehrerer Elektroden bzw. Elektroden-Arrays können die EMG-Signalstärken und -richtungen in Abhängigkeit des Ortes ermittelt werden.

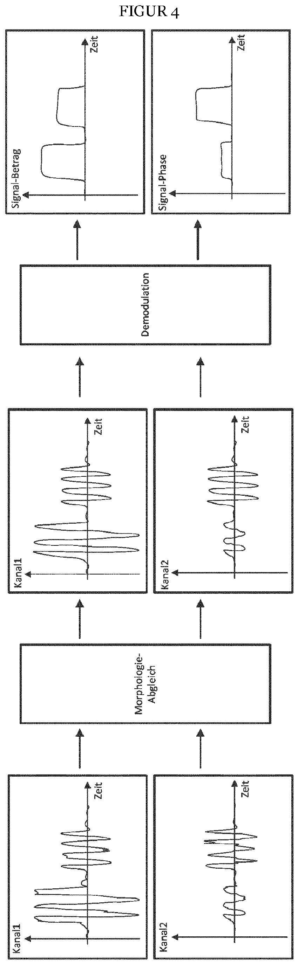

Die zweite Möglichkeit der Auswertung vergleicht die erfassten Zeitsignale noch vor der Bestimmung der gemittelten Signalstärken. Dazu werden im ersten Schritt die Morphologie beider erfasster Signale miteinander verglichen und extrahiert. Erst im Anschluss werden die Amplituden gleicher Morphologien miteinander verglichen und so die Ausbreitungsrichtung des EMG-Signals mittels Demodulation bestimmt, wie exemplarisch in Figur 4 gezeigt.

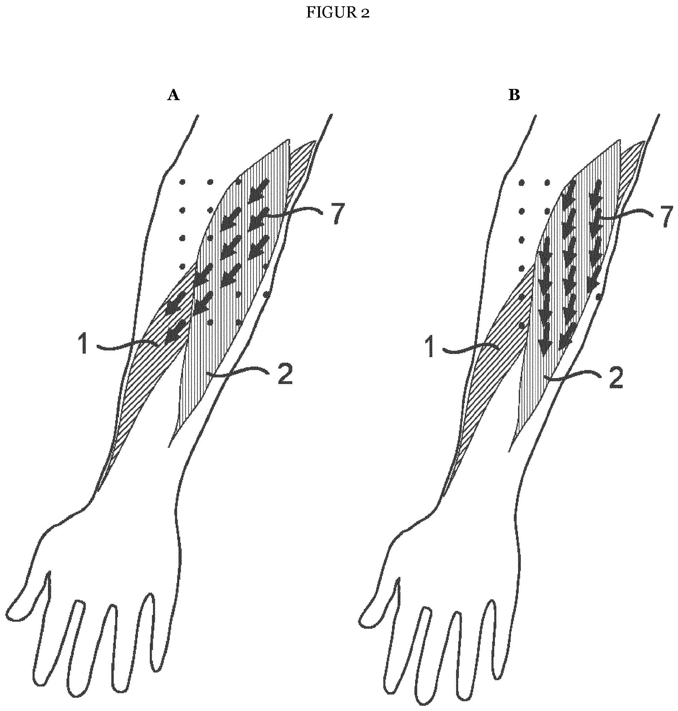

Wird dieses Verfahren nicht nur für eine Position durchgeführt, sondern mithilfe von Elektrodenarrays auch räumlich aufgelöst, so kann auf die Messoberfläche ein Vektorfeld projiziert werden, wie es in Figur 2 illustriert ist. Die Längen der eingetragenen Vektoren (7) repräsentieren in dieser Darstellungsform die Signalstärken und die Richtungen der Vektoren zeigen in die Ausbreitungsrichtung des EMG-Signals. Diese Darstellung wird im Rahmen dieser Erfindung hergenommen und mit der bekannten Physiologie der jeweils betrachteten Körperregion verglichen. Somit ist die Erfindung anwendbar auf Messungen an Einzelpositionen oder verteilte Messbereiche. Zudem können nicht nur 2-dimensionale Vektoren erzeugt werden, sondern durch entsprechende Elektroden-Applizierungen und Kanal-Konfigurationen auch 3-dimensionale Vektoren innerhalb des Gewebes.

Eine weitere Anwendung neben dem Vergleichen des vermessenen Vektorfeldes mit bekannten Gewebegeometrien bzw. Muskellagen ist das Erkennen bzw. Erlernen der geometrischen Begebenheiten. Ist das zuvor genannte Vorwissen nicht vorhanden oder steht im Widerspruch zu den Messungen, so können sinnvolle/zusammenhängende wiederauftretende Vektorfelder detektiert werden und so die Lage des Elektrodenarrays erkennen. Die gemessenen Signale werden nach der Erkennung entsprechend so transformiert, dass sie wieder mit den angenommenen Geometrien übereinstimmen und Zuordnungen zu Muskeln durchgeführt werden können.

**Beispiel 2: Ansteuerung einer Handprothese**

Es ist essentiell, dass Prothesen sicher angesteuert werden. Das typische Verfahren zur Ansteuerung von Prothesen ist die Elektromyographie, also die Messung von Muskelaktionspotentialen mittels differenzieller Spannungsmessung. Problematisch ist, dass oftmals nicht unterschieden werden kann, ob es sich bei dem aufgezeichneten Signal tatsächlich um ein EMG handelt oder ob es Störspannungen sind. Die zusätzliche Bestimmung der Ausbreitungsrichtung des gemessenen Signals ermöglicht Rückschlüsse auf die Plausibilität und erhöht somit die Störsicherheit.

Bezugnehmend auf die Figur 1 wird eine exemplarische geometrische Überlagerung von Muskeln im Unterarm veranschaulicht. Eine derartige geometrische Überlagerung macht es problematisch, dass oftmals nicht unterschieden werden kann, ob es sich bei dem aufgezeichneten Signal tatsächlich um ein EMG handelt oder ob es lediglich Störspannungen sind. Zudem ist es schwer analysierbar, welcher der beiden Muskeln im Unterarm, wie sie beispielhaft in Figur 1A gezeigt sind, für das Signal verantwortlich sind.

Die Ausbreitungsrichtung eines EMG-Signals soll daher mittels der 2-kanaligen differentiellen Spannungsmessung erfindungsgemäß bestimmt werden, wobei ein exemplarischer Messansatz zur Bestimmung von Signalstärke und Ausbreitungsrichtung eines EMG-Signals in Figur 1B gezeigt ist.

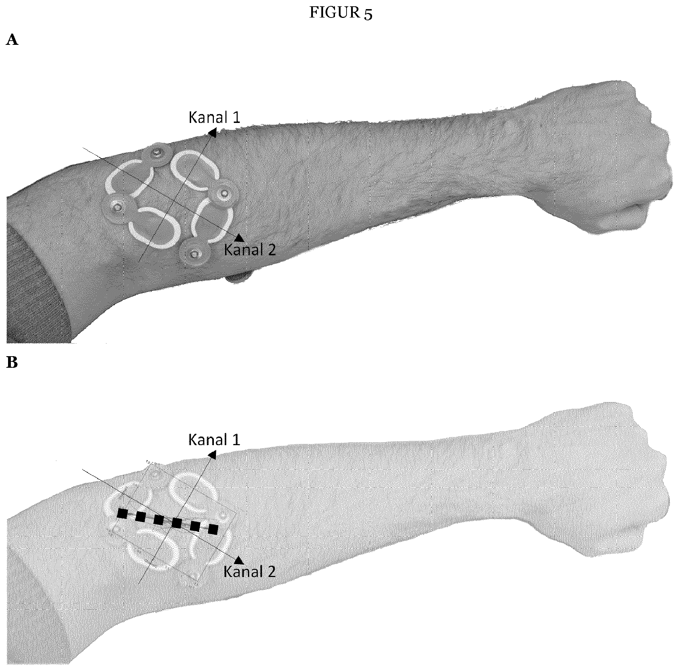

Bezugnehmend auf die Figur 5 wird die einfachste und intuitivste Anordnung der erfindungsgemäßen 2-kanaligen differentiellen Spannungsmessung beschrieben, welche die orthogonale Anordnung beider Kanäle mit je 2 äquidistanten Elektroden zur differentiellen Spannungsmessung ist. In der beispielhaften Anordnung der Elektroden, wie sie in Figur 5 gezeigt sind, befindet sich auf der Unterseite des Arms des Subjekts bzw. des Probanden eine zusätzliche Referenzelektrode zum schaltungstechnischen Potentialausgleich, was typisch beim EMG ist.

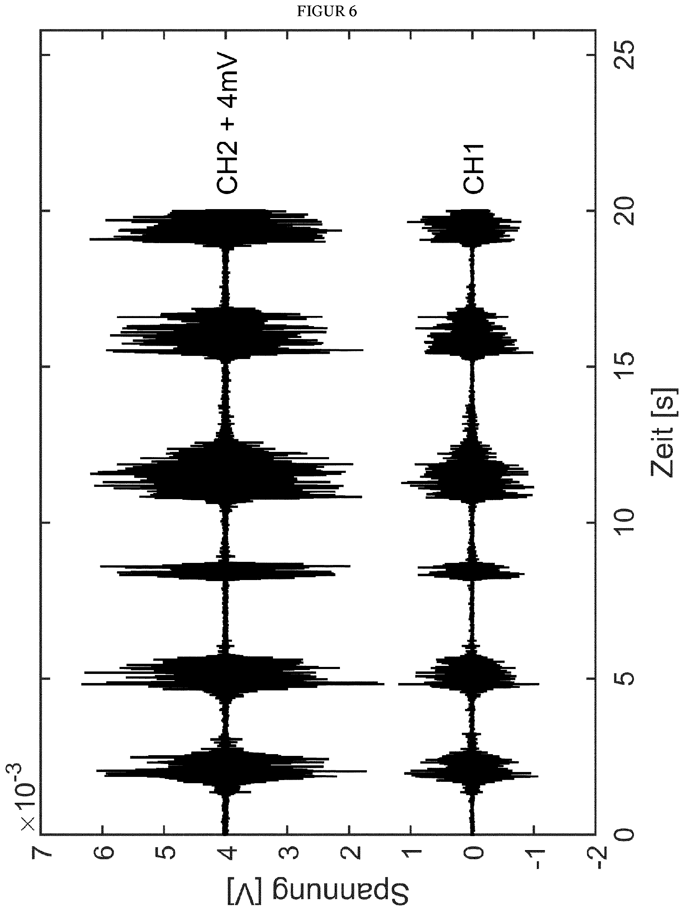

Bezugnehmend auf die Figur 6 sind die zeitkontinuierlich abgeleiteten differentiellen Spannungen beider Kanäle für eine Dauer von 20 Sekunden dargestellt. Der Proband hat im vorliegenden Beispiel innerhalb dieser Zeit (20 Sekunden) sechsmal sein Handgelenk durch Muskelkontraktion angewinkelt. Diese Perioden starker Signalamplituden sind in beiden Kanälen, welche als CH1 (Kanal 1) und CH2 (Kanal 2) gekennzeichnet sind, gut zu erkennen.

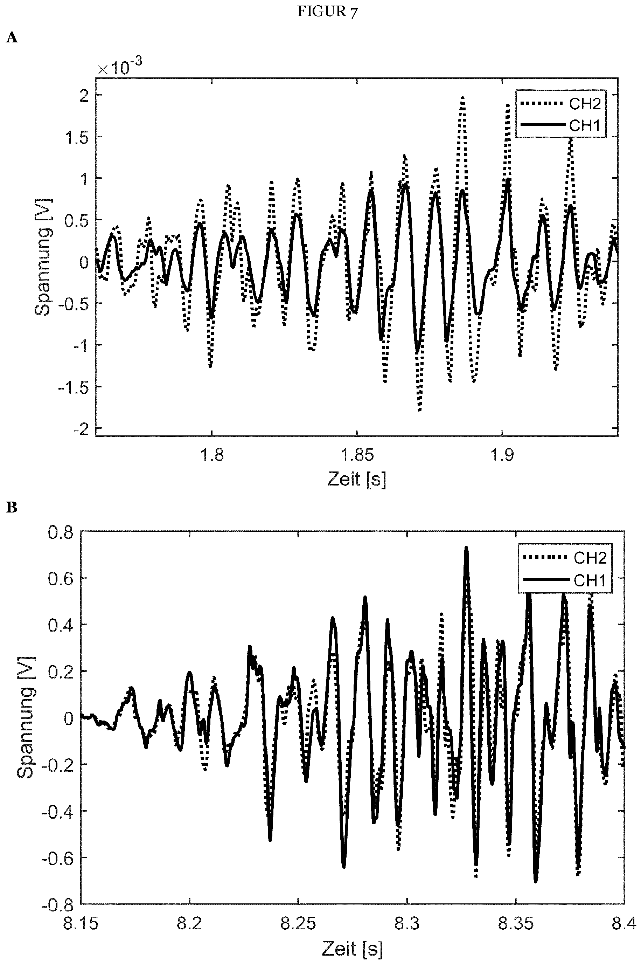

Unter Bezugnahme auf die Figur 7 wird eine Vergrößerung eines Zeitabschnitts aus Figur 6 gezeigt. In dieser Vergrößerung des Zeitabschnitts wird sichtbar, dass in einem hohen Maße Ähnlichkeiten zwischen den beiden Signalformen bestehen und sich diese fast ausschließlich in ihrer Amplitude unterscheiden. Bezugnehmend auf die Figur 7B werden diese Ähnlichkeiten noch deutlicher, wobei die in der Figur 7B gezeigten Signale vorab normiert wurden.

Durch unterschiedliche Verfahren der Signalverarbeitung können sich die sich ähnelnden Signalanteile dann für die weiteren Schritte extrahiert und die Unterschiede entfernt werden. Im einfachsten Falle können mittels Frequenzfilter Spektralanteile entfernt werden, die besonders unterschiedlich sind.

Durch die im vorliegenden Beispiel einfache orthogonale Elektrodenanordnung können dann die beiden Signalamplituden unmittelbar in einen Betrag und in eine Phase des Signalvektors umgerechnet werden, wie es von komplexen Zahlen bekannt ist:

$$|EMG| = \sqrt{Kanal1^2 + Kanal2^2}|$$

bzw.

$$\varphi = arctan2(Kanal1, Kanal2)$$

Unter Bezugnahme auf die Figur 4 wird die Signalverarbeitung zur Bestimmung von Betrag und Phase bzw. Ausbreitungsrichtung des detektierten EMG-Signals anhand des erfindungsgemäßen Verfahrens deutlich.

Bezugnehmend auf die Figur 5B wird deutlich, dass man den berechneten Vektor (der als schwarze gestrichelte Linie in Figur 5B gezeigt ist) gemäß des von den Elektroden aufgespannten Koordinatensystems auf die Abbildung aus Figur 5A legen kann. Dadurch zeigt dieser die Ausbreitungsrichtung des EMG-Signals, welches mit der Muskelfaserrichtung des darunterliegenden, angespannten Muskels übereinstimmt.

Weitere erfindungsgemäß bestimmte EMG-Vektorfelder werden beispielhaft in Figur 2 gezeigt, wobei das Vektorfeld in Figur 2A bei Kontraktion von Muskel 1 erzeugt wird. Dahingegen wird das Vektorfeld in Figur 2B bei Kontraktion von Muskel 2 erzeugt.

Die in der vorstehenden Beschreibung, den Figuren und den Ansprüchen offenbarten Merkmale können sowohl einzeln als auch in beliebiger Kombination für die Realisierung der Erfindung in den verschiedenen Ausgestaltungen von Bedeutung sein.

**Patentansprüche**

1. Ein Verfahren zur Separation von Nutz- und Störsignalen in einer elektromyografischen (EMG)-Messung und/oder zur Bestimmung des Signal-verursachenden Muskels in einer elektrisch aktiven Region an Muskeln, die folgenden Schritte umfassend:

(a) Anbringen von mindestens drei, vorzugsweise vier, fünf oder mehr, Elektroden auf unterschiedlichen Positionen auf mindestens zwei unterschiedlichen Achsen auf der Hautoberfläche und/oder in mindestens einem Muskel eines Subjekts, wobei die mindestens zwei unterschiedlichen Achsen eine mindestens erste und eine mindestens zweite Achse sind;

(b) Detektieren von mindestens einem EMG-Signal, das die myoelektrische Aktivität einer elektrisch aktiven Region an Muskeln des Subjekts beschreibt, mit den mindestens drei Elektroden;

(c) Messen der Spannung entlang der mindestens ersten und der mindestens zweiten Achse;

(d) Bestimmen einer muskulären Ausbreitungsrichtung des mindestens einem EMG-Signals, und

(e) Optional, Visualisieren und/oder Anzeigen der muskulären Ausbreitungsrichtung des mindestens einem EMG-Signals als mindestens ein Vektor.

2. Das Verfahren nach Anspruch 1, wobei die muskuläre Ausbreitungsrichtung des mindestens einem EMG-Signals in Schritt (d) des Verfahrens bestimmt wird durch:

(i) Demodulation des detektierten mindestens einem EMG-Signals durch Anwenden einer Mittelungsfunktion auf mindestens eine Signalamplitude der Spannung entlang der mindestens ersten Achse und mindestens eine Signalamplitude der Spannung entlang der mindestens zweiten Achse und dadurch Erzeugen einer gemittelten Signalamplitude der Spannung entlang der mindestens ersten Achse und einer gemittelten Signalamplitude der Spannung entlang der mindestens zweiten Achse, und

(ii) Bestimmen der muskulären Ausbreitungsrichtung des Nutzsignals durch einen Abgleich der gemittelten Signalamplituden.

3. Das Verfahren nach Anspruch 1, wobei die muskuläre Ausbreitungsrichtung des mindestens einem EMG-Signals in Schritt (d) des Verfahrens bestimmt wird durch:

(i) Extrahieren der Morphologie mindestens eines Signalabschnitts der Spannung entlang der mindestens ersten Achse und mindestens eines Signalabschnitts der Spannung entlang der mindestens zweiten Achse;

(ii) Vergleichen der Morphologie des mindestens einen Signalabschnitts der Spannung entlang der mindestens ersten Achse und des mindestens einen Signalabschnitts der Spannung entlang der mindestens zweiten Achse;

(iii) Auslegen und/oder Definieren mindestens eines Anteils des Signalabschnitts entlang der mindestens ersten Achse und eines Anteils des Signalabschnitts entlang der mindestens zweiten Achse, die einen Anteil an einer Ähnlichkeit aufweisen, als ein Nutzsignal, und

(iv) Bestimmen der muskulären Ausbreitungsrichtung des Nutzsignals mittels Demodulation.

**4.** Das Verfahren nach einem der Ansprüche 1 bis 3, wobei die muskuläre Ausbreitungsrichtung des mindestens einem EMG-Signals, und/oder der Vektor, mit mindestens einer Referenzmorphologie von einem Gewebe und/oder einer Muskellage verglichen wird, dadurch Bestimmung des Signal-verursachenden Muskels in einer elektrisch aktiven Region an Muskeln und/oder dadurch automatisiertes Erlernen der Platzierung der mindestens drei Elektroden.

**5.** Das Verfahren nach einem der Ansprüche 1 bis 4, wobei die mindestens drei Elektroden auf unterschiedlichen Positionen oberhalb von mindestens zwei übereinanderliegenden Muskeln auf der Hautoberfläche eines Subjekts in Schritt a) des Verfahrens angebracht werden.

**6.** Das Verfahren nach einem der Ansprüche 1 bis 5, wobei die Elektroden Oberflächenelektroden und/oder Nadelelektroden sind, und wobei die Elektroden vorzugsweise Oberflächenelektroden sind.

**7.** Das Verfahren nach einem der Ansprüche 1 bis 6, wobei die Elektroden äquidistante Elektroden sind und/oder die Elektroden auf orthogonalen Achsen positioniert sind.

**8.** Das Verfahren nach einem der Ansprüche 1 bis 7, wobei das Verfahren zusätzlich die Schritte umfasst:

(f) Messen einer elektrischen Bioimpedanz und/oder einer Impedanzmyographie (EIM), und optional Vergleichen des mindestens einem EMG-Signals mit der geometrischen Verteilung und/oder der zeitlichen Änderung der elektrischen Bioimpedanz und/oder der Impedanzmyographie (EIM), und/oder

(g) Extrahieren eines Mechanomyogramms (MMG)-Signals von einem oder mehreren Vibrationssensoren als Reaktion auf eine Oberflächenvibration eines Muskels des Subjekts, und/oder

(h) Rückmelden an das Subjekt oder den Benutzer, welcher Muskel kontrahiert ist, und/oder

(i) Überwachen, insbesondere digitales Überwachen, eines Trainings von mindestens einem Muskel, und/oder

(j) Erkennen und/oder Unterdrücken von Störsignalen von mindestens einem anderen Muskel in der elektrisch aktiven Region an Muskeln, beispielsweise dem Signal des Herzmuskels.

**9.** Eine Verwendung eines Verfahrens nach einem der Ansprüche 1 bis 8 zur EMG-Diagnostik von mindestens einem Muskel, insbesondere zur EMG-Diagnostik von mindestens zwei überlagernden Muskeln, zur Ansteuerung von Prothesen, zum Training von Muskeln, insbesondere zum Reha-Training und/oder zum Fitness-Training, oder zur Steuerung von Computer-Programmen und/oder Computer-Spielen.

**10.** Ein Computerimplementiertes Verfahren, umfassend den Schritt:

(a) Bestimmen einer muskulären Ausbreitungsrichtung mindestens eines EMG-Signals.

**11.** Eine Vorrichtung zur Datenverarbeitung, umfassend Mittel zur Ausführung des Schritts (a) des Verfahrens nach Anspruch 10.

**12.** Ein Computerprogrammprodukt, umfassend Befehle, die bei der Ausführung des Programms durch einen Computer diesen veranlassen, den Schritt (a) des Verfahrens nach Anspruch 10 auszuführen.

**13.** Ein Computerlesbares Speichermedium, umfassend Befehle, die bei der Ausführung durch einen Computer diesen veranlassen, den Schritt (a) des Verfahrens nach Anspruch 10 auszuführen.

FIGUR 1

FIGUR 2

A
B

FIGUR 3

FIGUR 4

FIGUR 5

A

Kanal 1

Kanal 2

B

Kanal 1

Kanal 2

FIGUR 6

FIGUR 7

**A**

**B**

Europäisches
Patentamt

European
Patent Office

Office européen
des brevets

# EUROPÄISCHER RECHERCHENBERICHT

**Nummer der Anmeldung**

**EP 22 20 0375**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | TOSHIKI KOSHIO ET AL: "Identification of surface and deep layer muscles activity by surface EMG", SICE ANNUAL CONFERENCE (SICE), 2012 PROCEEDINGS OF, IEEE, 20. August 2012 (2012-08-20), Seiten 1816-1821, XP032260025, ISBN: 978-1-4673-2259-1 | 1,3-13 | INV. A61B5/397 ADD. A61B5/00 |
| A | * das ganze Dokument * * insbesondere: * * Zusammenfassung * * Abbildungen 1-3,7,8 * * Abschnitte 1, 2.1, 2.2, 2.3, 2.4, 3 * ----- | 2 | |
| X | TOSHIKI KOSHIO ET AL: "Estimation of surface and deep layer muscle activity using electrode array", SYSTEM INTEGRATION (SII), 2012 IEEE/SICE INTERNATIONAL SYMPOSIUM ON, IEEE, 16. Dezember 2012 (2012-12-16), Seiten 487-491, XP032323461, DOI: 10.1109/SII.2012.6427288 ISBN: 978-1-4673-1496-1 | 1,3-13 | |
| A | * das ganze Dokument * * insbesondere: * * Zusammenfassung * * Abbildungen 1,6,7 * * Abschnitte II, III * ----- | 2 | RECHERCHIERTE SACHGEBIETE (IPC) A61B |

Der vorliegende Recherchenbericht wurde für alle Patentansprüche erstellt

| Recherchenort | Abschlußdatum der Recherche | Prüfer |
|---|---|---|
| Den Haag | 2. Februar 2023 | Albrecht, Ronald |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

........................................................................
& : Mitglied der gleichen Patentfamilie, übereinstimmendes Dokument

EPO FORM 1503 03.82 (P04C03)